# EUROPÄISCHE PATENTANMELDUNG

(11) **EP 0 721 937 A2**
(43) Veröffentlichungstag der Anmeldung: **17.07.1996**
(21) Anmeldenummer: 95120505.3
(22) Anmeldetag: 23.12.1995
(51) Int. Cl.: C07C 229/16, C07C 227/16, C08G 59/52

(54) **(Meth)Acrylsäure-tert.-butylester-Gruppen enthaltende Polyamine**

(30) Priorität: 10.01.1995 DE 19500427
(71) Anmelder: HÜLS AKTIENGESELLSCHAFT, D-45764 Marl (DE)
(72) Erfinder: Wolf, Elmar, Dr., D-45661 Recklinghausen (DE); Schleimer, Bernhard, D-45770 Marl (DE)

(57) **Zusammenfassung**

(Meth)Acrylsäure-tert.-butylester-Gruppen enthaltende Polyamine mit der allgemeinen Formel I 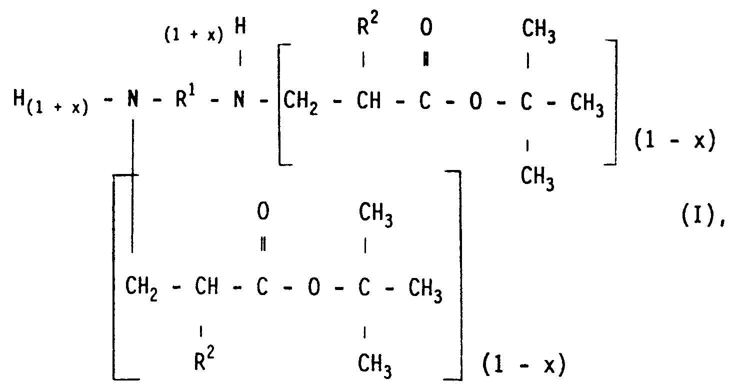
wobei R¹ ein (Cyclo)-alkylenrest mit 2 - 14 C-Atomen darstellt, der gegebenenfalls mit 1 - 3 CH₃- bzw. C₂H₅-Gruppen substituiert sein kann, und 1 - 3 CH₂-Gruppen durch Sauerstoff oder -NH-, -N-CH₃-, 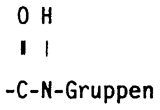
substituiert sein können, R² gleich Wasserstoff oder Methyl bedeutet und x eine Zahl zwischen 1 und 0 sein kann, mit der Maßgabe, daß die Summe 2 (1 - x) ≧ 0,1 betragen muß.

## Beschreibung

Gegenstand der vorliegenden Erfindung sind (Meth)Acrylsäure-tert.-butylester-Gruppen enthaltende Polyamine, sowie ein Verfahren zu ihrer Herstellung.

Bei den zur Härtung von Epoxidharzen eingesetzten Polyaminen handelt es sich um überwiegend (cyclo)aliphatische Polyamine. Die mit diesen Polyaminen gehärteten Epoxid-Harze zeichnen sich in der Praxis durch eine Reihe von erwünschten Eigenschaften aus, wie z. B. gute Haftung auf allen möglichen Substraten, gute Lösungsmittelbeständigkeit und hohe Resistenz gegenüber Chemikalieneinwirkung. In manchen Fällen hat es sich als vorteilhaft erwiesen, wenn zur Härtung von Epoxid-Harzen cyanethylierte Polyamine eingesetzt wurden. So werden in der DE-AS 10 34 856 härtbare Massen mit verbesserter Schlagfestigkeit (im ausgehärteten Zustand) beschrieben, die aus einem Glycidylpolyether und eine: Härtungsmittel, das durch Reaktion eines aliphatischen Polyamins mit Acrylnitril erhalten wurde, bestehen.

In der DE-PS 21 64 099 werden cyanethylierte aliphatische Polyamine, spez. Diamine beschrieben, die im Gemisch mit Epoxidverbindungen, die mehr als ein Äquivalent Epoxidgruppen pro Mol enthalten, insbesondere in lösungsmittelfreien Gemischen eine längere Topfzeit haben und mit klebfreier Oberfläche aushärten. In der DE-OS 24 60 305 werden Acrylnitril-Addukte von cycloaliphitischen Diaminen beansprucht, die in Kombination mit flüssigen Epoxidharzen spannungsarme Formstoffe mit geringem Schrumpf und geringer Rißanfälligkeit ergeben. In der USP 3 478 081 werden als Härter für Epoxidharze cyanethylierte Bis-(aminoalkyl)-cyclohexane genannt, welche zu Formstoffen mit guter Flexibilität und hoher Wärmebeständigkeit führen. Ein großer Nachteil dieser cyanethylierten Polyamine liegt in der Handhabung des cancerogenen Acrylnitrils bei der Herstellung der Acrylnitril-Polyamin-Addukte. Eine einfache Substitution des Acrylnitrils durch die nicht cancerogenen Acrylsäureester bei der Addition mit Polyaminen ist nicht möglich, da die Addukte aus Acrylsäureestern und Polyaminen nicht lagerstabil sind. Es tritt bereits bei Raumtemperatur eine langsame Amidierung der Estergruppen ein.

Überraschenderweise wurde nun gefunden, daß Estergruppen enthaltende Polyamine lagerstabil sind, wenn diese Reste von (Meth)Acrylsäure-tert.-butylester enthalten.

Gegenstand der vorliegenden Erfindung sind somit (Meth)Acrylsäure-tert.-butylester-Gruppen enthaltende Polyamine mit der allgemeinen Formel I 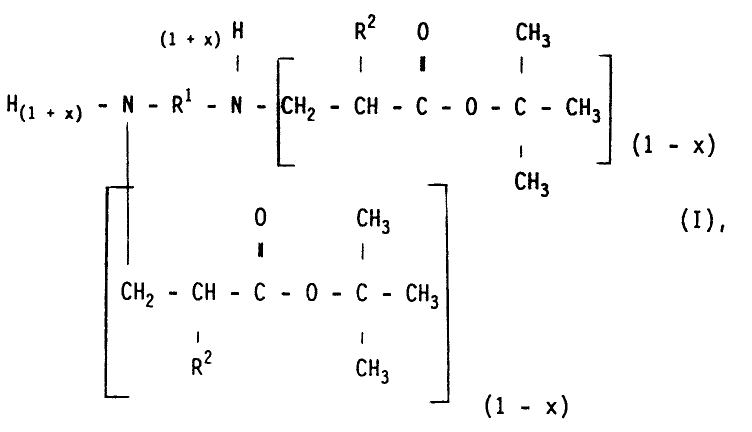
wobei R¹ ein (Cyclo)-alkylenrest mit 2 - 14 C-Atomen darstellt, der gegebenenfalls mit 1 - 3 CH₃- bzw. C₂H₅-Gruppen substituiert sein kann, und 1 - 3 CH₂-Gruppen durch Sauerstoff oder -NH-, -N-CH₃-, 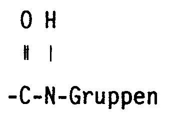
substituiert sein können, R² gleich Wasserstoff oder Methyl bedeutet und x eine Zahl zwischen 1 und 0 sein kann, mit der Maßgabe, daß die Summe 2 (1 - x) ≧ 0,1 betragen muß.

Bevorzugt werden Polyamine wobei R¹ gleich 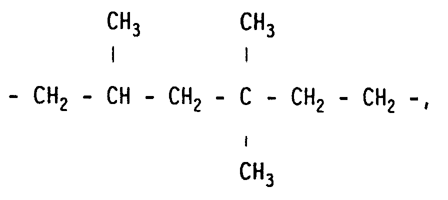 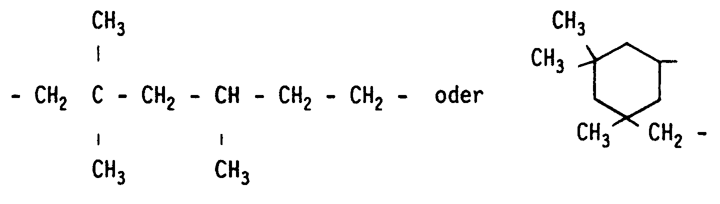
bedeutet.

Die erfindungsgemäßen Verbindungen sind charakterisiert durch einen Gehalt an (Meth)Acrylsäure-tert.-butylester von 0,1 - 2 mol (pro mol Polyamin) und einen basischen Amingehalt von 3 - 9 mmol NH₂/g. Ihre Viskosität bei Raumtemperatur liegt bei 100 - 10 000 mPa·s.

Ein weiterer Gegenstand der vorliegenden Erfindung ist ein Verfahren zur Herstellung der erfindungsgemäßen Verbindungen, dadurch gekennzeichnet, daß Polyamine mit (Meth)Acrylsäure-tert.-butylester entsprechend folgender Reaktionsgleichung umgesetzt werden. 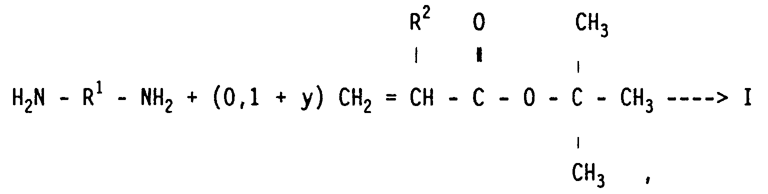
wobei R¹ und R² die obengenannte Bedeutung haben und y = 0 - 1,9 bedeutet.

Bei den im Sinne der vorliegenden Erfindung einzusetzenden Polyaminen handelt es sich um (cyclo)aliphatische Polyamine, wie z. B. 1.4-Diaminobutan, Neopentandiamin, 2-Methylpentamethylendiamin, 5-Methyl-nonamethylendiamin, 4.7-Dioxadecan-1.10-diamin, 4.9-Dioxadodecan-1.12-diamin, 4.7.10-Trioxatridecan-1.13-diamin, 2.2.4(2.4.4)-Trimethylhexamethylendiamin (TMD), Nonadecandiamin, Bis-(3-aminopropyl)-methylamin, Bis-Hexamethylentriamin, 4.4'-Diaminodicyclohexylmethan, 3.3'-Dimethyl-4.4'-diamino-dicyclohexylmethan, 2.2-Bis-(4-aminocyclohexyl)-propan, Isophorondiamin (IPD), 1.2-Diaminocyclohexan, m-Xylylendiamin.

Nach dem erfindungsgemäßen Verfahren wird zu dem auf 20 - 80 °C erhitzten Polyamin der (Meth)Acrylsäure-tert.-butylester unter intensiver Rührung so zudosiert, daß die Temperatur des Reaktionsgemisches nicht über 90 °C steigt. Nach beendigter Zugabe des (Meth)Acrylsäure-tert.-butylesters wird das Reaktionsgemisch zur Vervollständigung der Umsetzung noch ca. 1 h bei 80 °C weiter erhitzt. Die so hergestellten Verbindungen enthalten grundsätzlich für alle Werte von y mit Ausnahme y = 1,9 nicht umgesetztes Polyamin, mono- sowie disubstituiertes Polyamin. In der Regel werden die erfindungsgemäßen Verbindungen ohne weitere Behandlung, d. h. Reinigung, weiterverarbeitet. In manchen Fällen hat es sich als zweckmäßig erwiesen, die Verbindungen weitgehend frei vom Ausgangspolyamin (H₂N-R-NH₂) herzustellen. Man geht dann so vor, daß das Polyamin mit (Meth)Acrylsäure-tert.-butylester im Molverhältnis (5-10):1 umgesetzt wird, und nach beendeter Reaktion das nicht umgesetzte Polyamin durch Dünnschichtdestillation bei 80 - 170 °C und bei 0,1 bis 0,01 mbar vom Reaktionsprodukt abtrennt. Das so hergestellte Polyamin enthält nur noch geringe Mengen nicht umgesetztes Polyamin. Die Konzentration an N.N'-disubstituiertem Polyamin hängt vom Molverhältnis der Reaktanden ab. Je höher der Überschuß an Polyaminen ist, desto geringer ist die Konzentration des disubstituierten Polyamins.

Die erfindungsgemäßen Verbindungen eignen sich sehr gut zur Härtung von Epoxid-Harzen, zu deren Verarbeitung extrem lange Topfzeiten benötigt werden. Ganz besonders sind sie zur Herstellung von wäßrigen 2K-EP-Systemen geeignet.

### Experimenteller Teil

### Beispiel 1

In einem 1-l-Dreihalskolben wurden bei 60 °C zu 316 Gew.T. TMD unter intensiver Rührung und N₂-Abdeckung 256 Gew.T. Acrylsäure-tert.-butylester so zudosiert, daß die Temperatur des Reaktionsgemisches nicht über 90 °C stieg. Nach beendigter Zugabe des Acrylsäure-tert.-butylesters wurde das Reaktionsgemisch noch ca. 1 h bei 80 °C weiter erhitzt. Das Reaktionsprodukt setzte sich aus 20 Mol-% TMD sowie disubstituiertem TMD (1 TMD + 2 mol Acrylsäure-tert.-butylester) und 60 Mol-% monosubstituier-tem TMD (1 TMD + 1 mol Acrylsäure-t-butylester) zusammen.

Der Amingehalt des Reaktionsproduktes veränderte sich beim Lagern (bei Raumtemperatur sowie 50 °C) nicht.

| | |
|---|---|
| Amingehalt mmol NH₂/g | = 6,91 |
| NH-aktiv ÄG | = 95 |
| Viskosität (25 °C) mPa·s | = 90 |

### Beispiel 2

340 Gew.T. IPD wurden mit 256 Gew.T. Acrylsäure-tert.-butylester analog zum Beispiel 1 umgesetzt.

| | |
|---|---|
| Amingehalt mmol NH₂/g | = 6,70 |
| NH-aktiv ÄG | = 99 |
| Viskosität (25 °C) mPa·s | = 370 |

Der Amingehalt des Reaktionsproduktes veränderte sich beim Lagern (bei Raumtemperatur sowie 50 °C) nicht.

### Beispiel 3

232 Gew.T. 2-Methylpentamethylendiamin (Dylek A) wurden mit 256 Gew.T. Acrylsäure-tert.-butylester entsprechend den im Beispiel 1 beschriebenen Reaktionsbedingungen umgesetzt.

| | |
|---|---|
| Amingehalt mmol NH₂/g | = 8,12 |
| NH-aktiv ÄG | = 81 |
| Viskosität (25 °C) mPa·s | = 70 |

### Beispiel 4

340 Gew.T. IPD wurden mit 512 Gew.T. Acrylsäure-tert.-butylester entsprechend den im Beispiel 1 beschriebenen Reaktionsbedingungen umgesetzt.

| | |
|---|---|
| Amingehalt mmol NH₂/g | = 4,65 |
| NH-aktiv ÄG | = 213 |
| Viskosität (25 °C) mPa·s | = 930 |

### Beispiel 5

158 Gew.T. TMD wurden mit 128 Gew.T. Acrylsäure-tert.-butylester entsprechend den im Beispiel 1 beschriebenen Reaktionsbedingungen umgesetzt. Nach beendeter Umsetzung wurde das nicht umgesetzte TMD durch Dünnschichtdestillation bei 80 °C/0,1 mbar vom Reaktionsprodukt, das aus 95 Mol-% mono- und 5 Mol-% disubstituiertem TMD bestand, abgetrennt.

| | |
|---|---|
| Amingehalt mmol NH₂/g | = 6,83 |
| NH-aktiv ÄG | = 99 |
| Viskosität (25 °C) mPa·s | = 105 |

### Vergleichsbeispiel I

158 Gew.T. TMD wurden mit 128 Gew.T. Acrylsäure-n-butylester analog zu den im Beispiel 1 aufgeführten Bedingungen umgesetzt. Das Reaktionsprodukt war nicht lagerstabil.

| | |
|---|---|
| Amingehalt mmol NH₂/g | = 6,816 am 1. Tag |
| | 6,23 nach 28 d |
| | 5,86 nach 42 d |
| | 5,05 nach 70 d |

Mit Abnahme des Amingehaltes, d. h. mit zunehmender Amidierung, stieg auch die Viskosität.

### Vergleichsbeispiel II

170 Gew.T. IPD wurden mit 86 Gew.T. Acrylsäuremethylester analog zu den im Beispiel 1 aufgeführten Bedingungen umgesetzt. Das Reaktionsprodukt war nicht lagerstabil.

| | |
|---|---|
| Amingehalt mmol NH₂/g | = 7,43 am 1. Tag |
| | 6,49 nach 30 d |
| | 5,78 nach 120 d |

## Patentansprüche

1. (Meth)Acrylsäure-tert.-butylester-Gruppen enthaltende Polyamine mit der allgemeinen Formel I 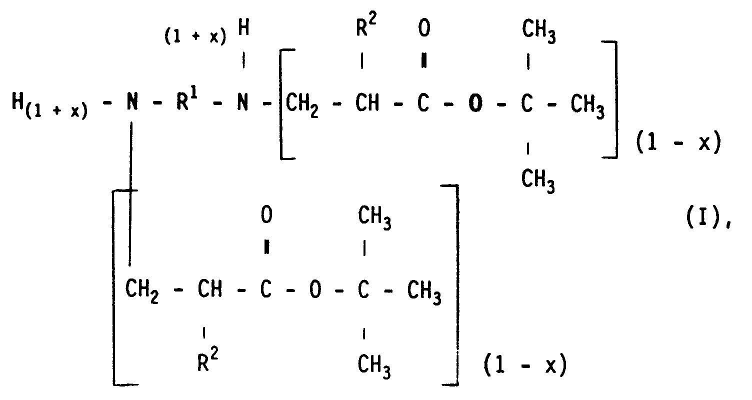 wobei R¹ ein (Cyclo)-alkylenrest mit 2 - 14 C-Atomen darstellt, der gegebenenfalls mit 1 - 3 CH₃- bzw. C₂H₅-Gruppen substituiert sein kann, und 1 - 3 CH₂-Gruppen durch Sauerstoff oder -NH-, -N-CH₃-, 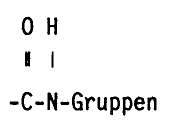 substituiert sein können, R² gleich Wasserstoff oder Methyl bedeutet und x eine Zahl zwischen 1 und 0 sein kann, mit der Maßgabe, daß die Summe 2 (1 - x) ≧ 0,1 betragen muß.

2. (Meth)Acrylsäure-tert.-butylester-Gruppen enthaltende Polyamine nach Anspruch 1,
dadurch gekennzeichnet,
daß R¹ gleich 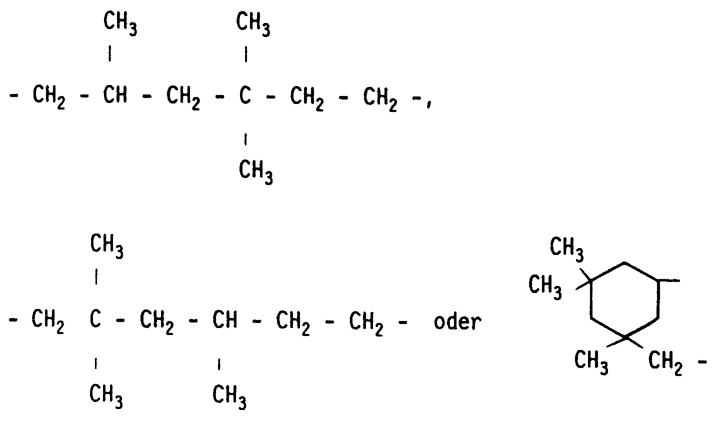 bedeutet.

3. Verfahren zur Herstellung von (Meth)Acrylsäure-tert.-butylester-Gruppen enthaltenden Polyaminen,
dadurch gekennzeichnet,
daß Polyamine der allgemeinen Formel
H₂N-R¹-NH₂ ,
wobei R¹ die gleiche Bedeutung wie im Anspruch 1 hat, mit (Meth)Acrylsäure-tert.-butylester im Molverhältnis 1 : (0,1 + y) bei 20 - 80 °C umgesetzt werden und y einen Wert zwischen 0 und 1,9 annehmen kann.

4. Verfahren zur Herstellung von monosubstituierten Polyaminen, die frei von Polyaminen sind,
dadurch gekennzeichnet,
daß in einem ersten Verfahrensschritt das Polyamin im Molverhältnis (5-10):1 mit (Meth)Acrylsäure-tert.-butylester umgesetzt wird, und anschließend die Abtrennung des nicht umgesetzten Polyamins durch eine Dünnschichtdestillation bei 80 - 170 °C und bei 0,1 bis 0,01 mbar erfolgt.

5. Verwendung der Polyamine nach den Ansprüchen 1 und 2 als Härterkomponente in Epoxid-Harzen.
